# EUROPEAN PATENT APPLICATION

(11) **EP 3 851 480 A1**
(43) Date of publication of application: **21.07.2021**
(21) Application number: 19860497.7
(22) Date of filing: 13.09.2019
(51) Int. Cl.: C08J 3/02, C07K 14/435, D01F 4/02, C12N 15/10, C12N 15/12

(54) **METHOD FOR PRODUCING FIBROIN SOLUTION AND METHOD FOR PRODUCING PROTEIN MOLDED BODY**

(30) Priority: 14.09.2018 JP 2018172728
(71) Applicant: Spiber Inc., Tsuruoka-shi, Yamagata 997-0052 (JP)
(72) Inventor: KUDO Hisahiro, Tsuruoka-shi, Yamagata 997-0052 (JP); ISHII Hideto, Tsuruoka-shi, Yamagata 997-0052 (JP); ADACHI Tatsuki, Tsuruoka-shi, Yamagata 997-0052 (JP); OKADA Ryoji, Tsuruoka-shi, Yamagata 997-0052 (JP)
(74) Representative: Handley, Matthew Edward
(86) International application number: PCT/JP2019/036241
(87) International publication number: WO 2020/054873

(57) **Abstract**

Disclosed is a method for manufacturing a fibroin solution including a step of forming a slurry containing a solvent-containing dissolving liquid and a fibroin-containing protein powder dispersed in the dissolving liquid by continuously introducing the protein powder to a thin film of the dissolving liquid while flowing the thin film; and a step of forming a fibroin solution by dissolving, in the dissolving liquid, the protein powder in the slurry.

## Description

### Technical Field

The present invention relates to a method for manufacturing a fibroin solution and a method for manufacturing a protein molded body.

### Background Art

Genetically modified fibroin is expected to be used as a material that can replace petroleum resources. For example, Patent Literature 1 discloses a technique for producing a fiber using, as a spinning raw liquid, a fibroin solution obtained by dissolving genetically modified fibroin in an appropriate solvent.

With regard to methods for manufacturing solutions of polymer materials, for example, Patent Literature 2 teaches a method involving dissolving an acrylonitrile-based polymer after producing a slurry in which the acrylonitrile-based polymer is dispersed. Patent Literature 2 also mentions that the slurry is obtained with generation of lumps suppressed by reducing the solubility of the acrylonitrile-based polymer by cooling the solvent. Patent Literature 3 discloses that the dissolving capacity of a solvent is reduced in a method for preparing a spinning solution for manufacturing acrylic fibers.

### Citation List

### Patent Literature

Patent Literature 1: JP 2014-129639 A
Patent Literature 2: JP 2015-078451 A
Patent Literature 3: JP 2015-132040 A

### Summary of Invention

### Technical Problem

It is expected that a high-concentration fibroin solution can be efficiently manufactured by a method involving dispersing a fibroin-containing powder in a solvent to obtain a slurry and then dissolving the powder in the solvent. However, this method may have a problem of generation of lumps in the step of forming the slurry.

However, a practical method for sufficiently suppressing generation of lumps in the step of forming a slurry in the manufacture of a fibroin solution is unknown so far. For fibroin, practical solvents are limited to high-melting-point solvents, such as dimethyl sulfoxide (DMSO) and formic acid, which makes it difficult to use the method in which the solubility is controlled by cooling the solvent as mentioned in Patent Literature 2. In addition, the use of the solvent together with a poor solvent for reducing the solubility in the solvent tends to easily generate a gel. There is also problem in which the use of a poor solvent requires heating at high temperature for dissolving fibroin in a solvent containing the poor solvent after forming a slurry. Heating at high temperature may degrade the physical properties of a protein molded body.

An object of one aspect of the present invention is to provide a method for effectively manufacturing a fibroin solution that may provide a protein molded body with good physical properties while suppressing generation of lumps in forming a slurry.

### Solution to Problem

In one aspect of the present invention, a method for manufacturing a fibroin solution includes:
a step of forming a slurry containing a solvent-containing dissolving liquid and a fibroin-containing protein powder dispersed in the dissolving liquid by continuously introducing the protein powder to a thin film of the dissolving liquid while flowing the thin film; and
a step of producing a fibroin solution by dissolving, in the dissolving liquid, the protein powder in the slurry.

In another aspect of the present invention, a method for manufacturing a protein molded body includes producing a fibroin solution by the above method and producing a fibroin-containing protein molded body by molding using the fibroin solution as a raw liquid for molding.

### Advantageous Effects of Invention

In a method according to one aspect of the present invention, a fibroin solution that may provide a protein molded body with good physical properties can be continuously and effectively manufactured with generation of lumps suppressed in forming a slurry.

### Brief Description of Drawings

Fig. 1 is a schematic view of one embodiment of a step of forming a slurry containing a protein powder.
Fig. 2 is a schematic view of one embodiment of the step of forming a slurry containing a protein powder.
Fig. 3 is a schematic view of one embodiment of the step of forming a slurry containing a protein powder.
Fig. 4 is a schematic view of one embodiment of a step of forming a fibroin solution from the slurry.

### Description of Embodiments

Some embodiments of the present invention will be described below in detail. However, the present invention is not limited to the following embodiments.

### (Method for Manufacturing Fibroin Solution)

One embodiment of a method for manufacturing a fibroin solution includes a dispersion step of forming a slurry by continuously introducing a fibroin-containing protein powder to a thin film of a dissolving liquid while flowing the thin film; and a dissolution step of producing a fibroin solution by dissolving, in the dissolving liquid, the protein powder in the slurry.

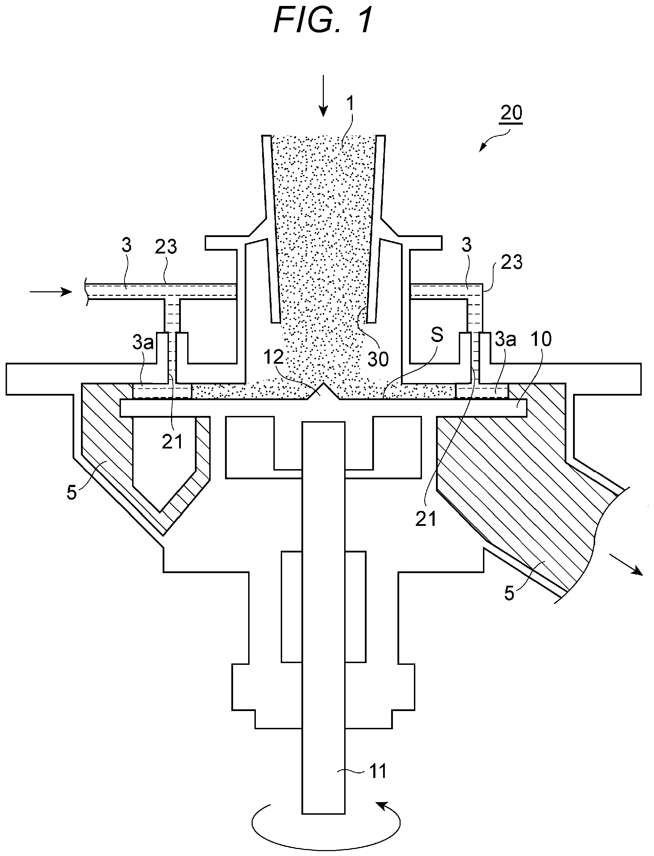

Fig. 1 is a schematic view of one embodiment of a step (dispersion step) of forming a slurry containing a protein powder. In the method shown in Fig. 1, a mixing device 20 including a plate-shaped rotary body 10 is used. The rotary body 10 has a rotation surface S and can be driven to rotate. With the rotary body 10 rotating, a protein powder 1 and a dissolving liquid 3 are each supplied onto the rotation surface S of the rotary body 10. The dissolving liquid 3 forms a flowing thin film 3a when the dissolving liquid 3 flows down in the radial direction on the rotation surface S. The flowing thin film 3a and the protein powder 1 are mixed on the rotation surface S. A slurry 5 formed as a result contains the dissolving liquid and the protein powder dispersed in the dissolving liquid. In Fig. 1, a clear boundary between the thin film 3a of the dissolving liquid and the slurry 5 is depicted for easy understanding. However, in general, a clear boundary is not formed between the thin film 3a and the slurry 5 because the thin film 3a to which the protein powder has been introduced gradually forms the slurry 5 while the thin film 3a flows.

The rotary body 10 has a disc shape. The rotation surface S is a circular plane. The rotation surface S has a conical controller 12 at its central portion. The controller 12 controls the flow of the powder. The shape of the rotary body is not limited to this. For example, the rotation surface may have a conical shape. In this case, the thin film of the dissolving liquid normally flows in a spiral manner. The rotary body may have a cylindrical shape. The rotation surface may have unevenness as desired. Like the embodiment in Fig. 1, the circular rotation surface S and the disc-shaped rotary body 10 having the controller 12 are advantageous in dispersion efficiency, processing efficiency, and device energy consumption. The rotary body 10 is driven to rotate by a shaft 11 connected to a driving device, such as a motor. The rotational speed of the rotary body 10 is not particularly limited, but may be, for example, 50 to 1000 rpm. The width of the rotation surface S is not particularly limited, but may be, for example, 50 to 1000 mm.

In the embodiment in Fig. 1, the dissolving liquid 3 moves in a feed pipe 23 connected to two dissolving liquid inlets 21 disposed adjacent to the rotation surface S. The dissolving liquid 3 is next supplied to the rotation surface S through the dissolving liquid inlets 21. The supplied dissolving liquid 3 forms the thin film 3a flowing on the rotation surface S. The formed thin film 3a of the dissolving liquid 3 immediately meets the protein powder 1 flowing on the rotation surface S from its central portion in the radial direction. The protein powder 1 is accordingly introduced to the flowing thin film 3a. The introduction of the protein powder 1 to the flowing thin film 3a of the dissolving liquid 3 allows the protein powder 1 to be readily uniformly dispersed without concentrating in a particular area. As a result, the protein powder 1 can be effectively dispersed in the dissolving liquid 3 with generation of lumps suppressed.

The dissolving liquid 3 contains a solvent for dissolving fibroin. With the dissolving liquid 3 being fed with a general-purpose pump, the flow rate of the dissolving liquid 3 may be measured and controlled. To prevent fluctuations in flow rate, the dissolving liquid 3 may be continuously supplied with a metering pump. Examples of the metering pump include gear pumps and uniaxial eccentric screw pumps. Gear pumps are superior in flow rate control accuracy. The flow rate of the dissolving liquid 3 may be, for example, 1 to 3000 kg/hr.

The solvent in the dissolving liquid 3 may contain, for example, at least one selected from the group consisting of hexafluoroisopropanol (HFIP), hexafluoroacetone (HFA), dimethyl sulfoxide (DMSO), N,N-dimethylformamide (DMF), and formic acid. From the viewpoint of fibroin solubility, the solvent may be dimethyl sulfoxide, formic acid, hexafluoroisopropanol (HFIP), or a mixed solvent containing a combination thereof.

The dissolving liquid 3 may further contain one or two or more inorganic salts dissolved in the solvent. Inorganic salts can promote the dissolution of fibroin. Examples of inorganic salts include inorganic salts composed of the following Lewis acid and Lewis base. Examples of the Lewis base include an oxo acid ion (nitrate ion, perchlorate ion, or the like), a metal oxo acid ion (permanganate ion or the like), a halide ion, a thiocyanate ion, and a cyanate ion. Examples of the Lewis acid include a metal ion such as an alkali metal ion or an alkaline earth metal ion, a polyatomic ion such as an ammonium ion, and a complex ion.

Examples of inorganic salts include lithium salts such as lithium chloride, lithium bromide, lithium iodide, lithium nitrate, lithium perchlorate, and lithium thiocyanate; calcium salts such as calcium chloride, calcium bromide, calcium iodide, calcium nitrate, calcium perchlorate, and calcium thiocyanate; iron salts such as iron chloride, iron bromide, iron iodide, iron nitrate, iron perchlorate, and iron thiocyanate; aluminum salts such as aluminum chloride, aluminum bromide, aluminum iodide, aluminum nitrate, aluminum perchlorate, and aluminum thiocyanate; potassium salts such as potassium chloride, potassium bromide, potassium iodide, potassium nitrate, potassium perchlorate, and potassium thiocyanate; sodium salts such as sodium chloride, sodium bromide, sodium iodide, sodium nitrate, sodium perchlorate, and sodium thiocyanate; zinc salts such as zinc chloride, zinc bromide, zinc iodide, zinc nitrate, zinc perchlorate, and zinc thiocyanate; magnesium salts such as magnesium chloride, magnesium bromide, magnesium iodide, magnesium nitrate, magnesium perchlorate, and magnesium thiocyanate; barium salts such as barium chloride, barium bromide, barium iodide, barium nitrate, barium perchlorate, and barium thiocyanate; and strontium salts such as strontium chloride, strontium bromide, strontium iodide, strontium nitrate, strontium perchlorate, and strontium thiocyanate. The inorganic salt may be lithium chloride, calcium chloride, or a combination thereof.

The content of the inorganic salt with respect to the total mass of the dissolving liquid may be 0.1% by mass or more, 1% by mass or more, 4% by mass or more, 7% by mass or more, 10% by mass or more, or 15% by mass or more, and may be 20% by mass or less, 16% by mass or less, 12% by mass or less, or 9% by mass or less. The content of the inorganic salt with respect to the total mass of the dissolving liquid may be 0.1% by mass or more and 20% by mass or less, 16% by mass or less, 12% by mass or less, or 9% by mass or less, may be 1% by mass or more and 20% by mass or less, 16% by mass or less, 12% by mass or less, or 9% by mass or less, may be 4% by mass or more and 20% by mass or less, 16% by mass or less, 12% by mass or less, or 9% by mass or less, may be 7% by mass or more and 20% by mass or less, 16% by mass or less, 12% by mass or less, or 9% by mass or less, may be 10% by mass or more and 20% by mass or less, 16% by mass or less, 12% by mass or less, or 9% by mass or less, or may be 15% by mass or more and 20% by mass or less, 16% by mass or less, 12% by mass or less, or 9% by mass or less.

By using, for example, a feeder, the protein powder 1 is continuously supplied toward a central portion of the rotation surface S through a powder inlet 30 disposed above the rotation surface S. The protein powder 1 is accordingly introduced to the thin film of the dissolving liquid 3 without coming into contact with an area wetted with the dissolving liquid. When the protein powder before being introduced to the thin film of the dissolving liquid comes into contact with a small amount of dissolving liquid on the rotary body, the protein powder adheres to the rotary body or the like, which may hinder continuous dispersion.

The feeder for supplying the protein powder 1 may be a metering feeder. The use of a metering feeder makes it easy to produce a fibroin solution having a constant concentration of fibroin. Fluctuations in fibroin concentration may significantly affect the quality of protein molded bodies such as protein fibers. The supply rate of the protein powder 1 may be, for example, 1 to 1000 kg/hr.

The shape of individual primary particles constituting the protein powder 1 is not particularly limited. The primary particles may be in the form of particles. If the primary particles have a small particle size, lumps tend to be generated. Even in such a case, generation of lumps can be suppressed according to this embodiment.

During the process for forming the slurry 5, heat may be generated. For this, the mixing device may be cooled, or the dissolving liquid 3 to be supplied may be cooled in advance. The mixing device can be cooled with, for example, a jacket surrounding the device. The cooling temperature of the jacket and the solvent may be -20°C or more and 30°C or less, -10°C or more and 20°C or less, 0°C or more and 15°C or less, or 5°C or more and 10°C or less.

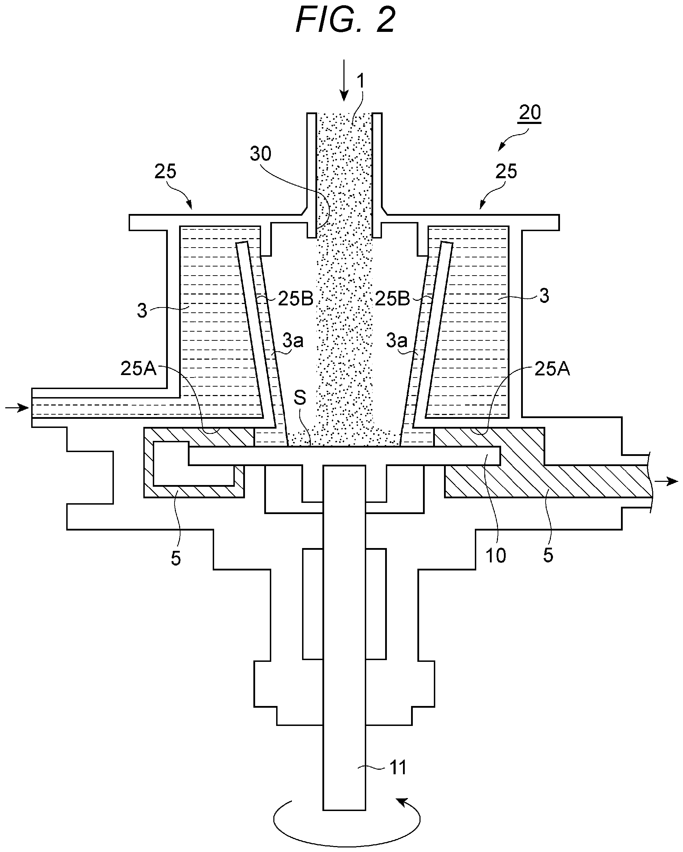

Fig. 2 is a schematic view of another embodiment of the step (dispersion step) of forming a slurry containing a protein powder. A mixing device 20 shown in Fig. 2 has an annular retention tank 25 disposed above a rotation surface S. The retention tank 25 has a bottom surface 25A and an inner circumferential surface 25B. The bottom surface 25A faces the rotation surface S. The inner circumferential surface 25B forms an opening above a central portion of the rotation surface S. A dissolving liquid 3 is retained in the retention tank 25, then flows down on the inner circumferential surface 25B from above the inner circumferential surface 25B while forming a thin film 3a, and is supplied onto the rotation surface S below the inner circumferential surface 25B. A protein powder 1 is continuously supplied toward a central portion of the rotation surface S through a powder inlet 30 disposed above the rotation surface S and meets the thin film of the dissolving liquid 3 on the rotation surface S at a position below the inner circumferential surface 25B.

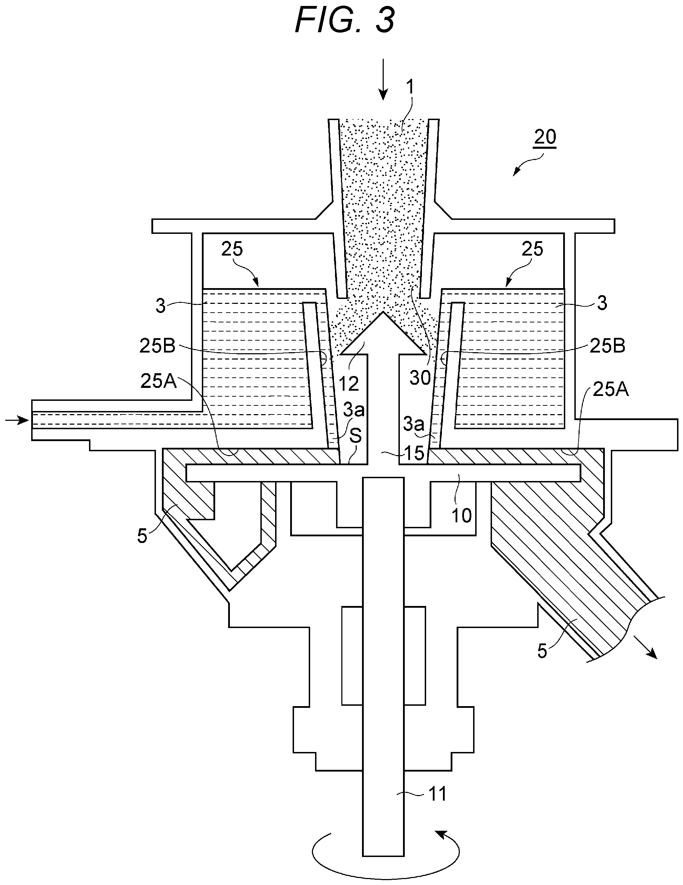

Fig. 3 is also a schematic view of another embodiment of the step (dispersion step) of forming a slurry containing a protein powder. A mixing device 20 shown in Fig. 3 has an annular retention tank 25 disposed above a rotation surface S. The retention tank 25 has a bottom surface 25A and an inner circumferential surface 25B. The bottom surface 25A faces the rotation surface S. The inner circumferential surface 25B forms an opening above a central portion of the rotation surface S. A dissolving liquid 3 is retained in the retention tank 25, then flows down on the inner circumferential surface 25B from above the inner circumferential surface 25B while forming a thin film 3a, and is supplied onto the rotation surface S below the inner circumferential surface 25B.

The mixing device 20 in Fig. 3 further has a columnar portion 15. The columnar portion 15 extends from a central portion of the rotation surface S toward a powder inlet 30. The columnar portion 15 has a conical controller 12 at its end. The controller 12 controls the flow of the protein powder 1. A protein powder 1 is continuously supplied toward the controller 12 through the powder inlet 30. The protein powder 1 flowing in directions changed by the controller 12 meets the thin film 3a flowing on the inner circumferential surface 25B before the protein powder 1 reaches the rotation surface S. The protein powder 1 is accordingly introduced to the thin film 3a on the rotation surface S of the dissolving liquid 3.

The other structures of the embodiments in Fig. 2 and Fig. 3 are the same as those of the embodiment in Fig. 1.

Examples of the mixing device used in the method illustrated above include a flow jet mixer (model MW-J-300) available from Funken Powtechs, Inc.

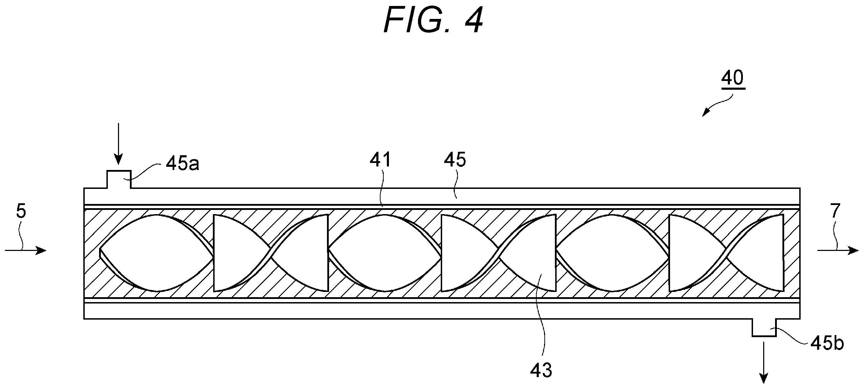

Fig. 4 is a schematic view of one embodiment of a step (dissolution step) of forming a fibroin solution from a slurry. An in-line mixer 40 shown in Fig. 4 has a pipe 41, a static mixer element 43 accommodated inside the pipe 41, and a jacket 45 covering the outer circumferential surface of the pipe 41. The jacket 45 has a heating medium inlet 45a and a heating medium outlet 45b. A heating medium that has been heated to a predetermined temperature flows from the heating medium inlet 45a to the heating medium outlet 45b. A slurry 5 passing through the in-line mixer 40 is heated accordingly.

The slurry 5 is introduced from one end of the pipe 41. The slurry 5 is stirred with the static mixer element 43 in the pipe 41 and heated with the heat transferred from the jacket 45. The protein powder in the slurry 5 is accordingly dissolved in the dissolving liquid to form a fibroin solution 7. The formed fibroin solution 7 is discharged from the other end of the pipe 41.

The temperature (heating temperature) of the heating medium introduced into the jacket 45 is adjusted such that the fibroin solution discharged from the in-line mixer reaches an intended temperature. The temperature of the fibroin solution 7 discharged from the in-line mixer 40 may be 20°C or more and 120°C or less, 30°C or more and 100°C or less, 40°C or more and 90°C or less, or 50°C or more and 80°C or less. If this temperature is below 20°C, more fibroin tends to remain undissolved. If this temperature is above 120°C, fibroin may thermally decompose.

The inner diameter and length of the pipe 41 of the in-line mixer 40 and the number of static mixer elements 43 are selected such that the protein powder dissolves well in the dissolving liquid. The time it takes for the slurry 5 to pass through the pipe 41, or the dissolution time, is 10 seconds or more and 300 seconds or less, 20 seconds or more and 200 seconds or less, 30 seconds or more and 120 seconds or less, or 40 seconds or more and 90 seconds or less. When the dissolution time is 10 seconds or more, it is easy to sufficiently dissolve the protein powder. A dissolution time of 300 seconds is normally adequate for sufficient dissolution. An exceedingly long dissolution time requires an unnecessarily huge device.

The method for dissolving, in the dissolving liquid, the protein powder in the slurry is not limited to the method using the in-line mixer illustrated in Fig. 4. For example, the protein powder may be dissolved in the dissolving liquid by heating in a stirring vessel, addition of a solvent, use of a mixed solvent, addition of a salt, or a combination thereof.

The method for manufacturing a fibroin solution may further include a step of removing insoluble matter by filtering the fibroin solution. The fibroin solution discharged from the in-line mixer in the dissolution step may be filtered through a filter disposed downstream of the in-line mixer. When the fibroin solution is filtered while it has high temperature, the pressure drop is small because of low viscosity of the solution.

The method for manufacturing a fibroin solution may further include a step of defoaming the fibroin solution. The fibroin solution discharged from the in-line mixer in the dissolution step may be defoamed by a defoaming device disposed downstream of the in-line mixer. When the fibroin solution is filtered while it has high temperature, the speed of moving bubbles is high because of low viscosity of the solution. Examples of the defoaming method include a method involving enclosing the fibroin solution inside a sealed container and depressurizing the sealed container, a method involving forming the fibroin solution into a thin film under reduced pressure, and a method involving centrifugal gas-liquid separation.

The method for manufacturing a fibroin solution may further include a step of cooling the fibroin solution under stirring in the in-line mixer. When the method for manufacturing a fibroin solution includes a step of removing insoluble matter from the fibroin solution and/or a step of defoaming the fibroin solution, the fibroin solution may be cooled after these steps. The temperature of the fibroin solution after cooling may be 0°C or more and 40°C or less. Although the viscosity of the fibroin solution greatly varies depending on temperature, the fibroin solution tends to be easily fed as long as the fibroin solution has a temperature of 10°C or more. As long as the fibroin solution has a temperature of 40°C or less, it is easy to sufficiently suppress decomposition of fibroin even when the fibroin solution is retained for a long period of time. Suppressing decomposition of fibroin provides a protein molded body having better physical properties. From the same viewpoint, the temperature of the fibroin solution after cooling may be 5°C or more and 30°C or less, 10°C or more and 25°C or less, or 15°C or more and 20°C or less.

The concentration of fibroin in the manufactured fibroin solution is not particularly limited, but may be, for example, 5% to 50% by mass, 10% to 50% by mass, 20% to 50% by mass, or 25% to 50% by mass based on the mass of the fibroin solution. The fibroin solution having a high concentration of fibroin can have better spinnability and, when being formed into a fiber being a protein molded body, enables drawing at a higher draw ratio. With a higher draw ratio, it is possible to obtain a fiber having high strength. Since lump generation and the amount of undissolved matter are small in the method according to this embodiment, it is easy to obtain a high-concentration fibroin solution. The manufactured fibroin solution may be directly subjected to the molding step or may be stored in a storage tank.

### (Method for Manufacturing Protein Molded Body)

A protein molded body containing fibroin can be manufactured by molding using, as a raw liquid for molding, the fibroin solution obtained by the method according to the embodiment. The manufactured protein molded body may be, for example, a film or fiber.

The manufactured protein molded body is a molded body containing fibroin as a main component. The proportion of fibroin in the total mass of the protein molded body may be 50% by mass or more, 60% by mass or more, 65% by mass or more, 70% by mass or more, 75% by mass or more, 80% by mass or more, or 90% by mass or more, or may be 100% by mass or less.

A protein fiber can be manufactured by spinning using the fibroin solution as a spinning raw liquid. The spinning step, which is an example of the molding step, will be described below.

The fibroin solution serving as a spinning raw liquid may further contain various additives as necessary. Examples of additives include plasticizers, leveling agents, cross-linking agents, crystal nucleating agents, antioxidants, ultraviolet absorbers, coloring agents, fillers, and synthetic resins. The content of the additives may be 0 parts by mass or more and 50 parts by mass or less with respect to 100 parts by mass of the total amount of fibroin in the spinning raw liquid.

In the spinning step, the fibroin solution is extruded from a spinneret and next formed into a fiber shape by removing the solvent from the fibroin solution. The fibroin solution may be fed to the spinneret from the storage tank by using a metering pump. The shape of the spinneret, the hole shape, the number of holes, and the like are not particularly limited and can be appropriately selected according to a desired fiber diameter and the number of filaments.

When the spinneret has a circular hole shape, the hole diameter may be 0.03 mm or more and 0.6 mm or less. With a hole diameter of 0.03 mm or more, it is possible to reduce the pressure drop and the equipment cost. With a hole diameter of 0.6 mm or less, the need of the drawing operation for reducing the fiber diameter is reduced, which makes it possible to reduce the possibility of drawing breakage between extrusion and take-up.

The temperature of the fibroin solution passing through the spinneret and the temperature of the spinneret are not particularly limited, and appropriately adjusted by, for example, the concentration and viscosity of fibroin in the fibroin solution and the type of solvent. The temperature of the fibroin solution passing through the spinneret may be 0°C to 70°C in order to prevent, for example, degradation of fibroin. The temperature of the fibroin solution passing through the spinneret may be below the boiling point of the solvent in order to reduce an increase in pressure caused by volatilization of the solvent and the possibility of generation of blockage in the pipe caused by solidification of the spinning raw liquid. This improves the process stability.

The method for removing the solvent of the fibroin solution extruded from the spinneret is not particularly limited. For example, the solvent may be volatilized by hot air in a gas phase. The solvent may be removed in a liquid phase containing a poor solvent that may be mixed with the solvent of the fibroin solution without dissolving fibroin. When the solvent of the fibroin solution is removed in a liquid phase, the fibroin solution may be extruded from the spinneret into the liquid phase through the gas phase or may be directly extruded into the liquid phase without passing through the gas phase.

A fiber formed by removal of the solvent may be drawn. The draw ratio based on the time point at which the formed fiber passes through the first take-up roller may be 2 times or more and 30 times or less, 15 times or less, or 10 times or less, may be 3 times or more and 30 times or less, 15 times or less, or 10 times or less, may be 4 times or more and 30 times or less, 15 times or less, or 10 times or less, may be 4 times or more and 30 times or less, 15 times or less, or 10 times or less, may be 5 times or more and 30 times or less, 15 times or less, or 10 times or less, or may be 6 times or more and 30 times or less, 15 times or less, or 10 times or less. The method according to this embodiment tends to enable the fibroin solution formed with generation of lumps suppressed to undergo spinning at higher draw ratio. The draw ratio is adjusted within a range in which desired fiber thickness and characteristics such as mechanical properties are obtained. Drawing may be performed once or may be performed stepwise several times. Drawing may be performed in liquid or may be performed after drying.

As necessary, an oil for providing bundle compactness, charge suppression, and lubricity may be applied to the formed protein fiber as desired before drying and winding. The type, amount, and the like of applied oil are not particularly limited and can be appropriately adjusted in consideration of, for example, application and handleability of fiber.

The formed protein fiber is dried by a usual method. Subsequently, the protein fiber may be wound by a winder. The winding conditions, such as suitable tension and contact pressure, in the winder are freely adjusted.

A fibroin film can be obtained by, for example, a method including subjecting the fibroin solution, which is used as a dope solution, to cast molding on the surface of a base member and drying the formed coating film and/or performing solvent removal.

The viscosity of the dope solution for manufacturing the fibroin film may be 15 to 80 cP (centipoise) or 20 to 70 cP. The concentration of fibroin may be 3% to 50% by mass, 3.5% to 35% by mass, or 4.2% to 15.8% by mass with respect to 100% by mass of the total amount of the dope solution.

The base member may be, for example, a resin substrate, a glass substrate, or a metal substrate. The base member may be a resin substrate since it is easy to peel a formed film. The resin substrate may be, for example, a polyethylene terephthalate (PET) film, a fluororesin film such as a polytetrafluoroethylene film, a polypropylene (PP) film, or a release film having a silicone compound immobilized on the surface of such a film. The base member may be a release film having a silicone compound immobilized in a PET film or on the surface of a PET film because the release film is stable against solvents such as HFIP and DMSO, the dope solution can stably undergo cast molding, and it is easy to peel a film after molding.

An example of cast molding includes casting a dope liquid onto the surface of a base member and adjusting the thickness of the coating film to a predetermined thickness by using a film thickness control means, such as an applicator, a knife coater, and a bar coater. The predetermined thickness is set such that the thickness of the coating film after drying and/or solvent removal is, for example, 1 to 1000 µm.

The drying and solvent removal of the coating film can be carried out by a dry method or a wet method. Examples of the dry method include vacuum drying, hot-air drying, and air drying. Examples of the wet method include immersion of the coating film in a solvent removal liquid. Examples of the solvent removal liquid include water, C1-C5 lower alcohols, such as methanol, ethanol, and 2-propanol, and a mixture of water and an alcohol. The temperature of the solvent removal liquid may be 0°C to 90°C.

After drying and/or solvent removal, the formed fibroin film may be uniaxially drawn or biaxially drawn in water. Biaxial drawing may be sequential drawing or simultaneous biaxial drawing. Multi-stage drawing of two or more stages may be performed. The draw ratio may be 1.01 to 6 times, or 1.05 to 4 times both in length and width. With the draw ratio within this range, it is easy to balance stress and strain. The drawing in water can be performed at a water temperature of, for example, 20°C to 90°C. The fibroin film after drawing may undergo heat setting under dry heating at 50°C to 200°C for 5 to 600 seconds. This heat setting provides a fibroin film having good dimensional stability at room temperature. In general, a uniaxially drawn film is a uniaxially oriented film, and a biaxially drawn film is a biaxially oriented film.

### (Fibroin)

Fibroin used for manufacturing a fibroin solution and a protein molded body may be natural fibroin or may be modified fibroin derived from natural fibroin. Fibroin may be fibroin manufactured from microorganisms or the like by genetic recombination technology, may be chemically synthesized fibroin, or may be purified naturally-occurring fibroin.

Fibroin may be, for example, one or more selected from the group consisting of silk fibroin, spider silk fibroin, and hornet silk fibroin. In particular, fibroin may be silk fibroin, spider silk fibroin, or a combination thereof. In combination of silk fibroin and spider silk fibroin, the proportion of silk fibroin is, for example, 40 parts by mass or less, 30 parts by mass or less, or 10 parts by mass or less with respect to 100 parts by mass of spider silk fibroin.

Silk fibroin may be sericin-removed silk fibroin, sericin-unremoved silk fibroin, or a combination thereof. Sericin-removed silk fibroin is produced by purifying silk fibroin by removing sericin covering silk fibroin, other fats, and the like. The purified silk fibroin may be a freeze-dried powder. Sericin-unremoved silk fibroin is unpurified silk fibroin from which sericin and the like have not been removed.

Spider silk fibroin may be natural spider silk fibroin, or modified spider silk fibroin (artificial spider silk fibroin) derived from natural spider silk fibroin.

Examples of natural spider silk fibroin include large spinneret dragline silk proteins, weft thread silk proteins, and minor ampullate gland silk proteins. Large spinneret dragline silk proteins have both high stress and stretchability because they have a repeated region consisting of a crystalline region and a noncrystalline region (also referred to as an amorphous region). Weft thread silk proteins have a characteristic of not having a crystalline region but having a repeated region consisting of a non-crystalline region. Weft thread silk proteins are inferior in stress as compared with large spinneret dragline silk proteins, but have high stretchability.

Large spinneret dragline silk proteins are also characterized by having excellent toughness because they are produced from the major ampullate gland. Examples of large spinneret dragline silk proteins include major ampullate spidroins MaSp1 and MaSp2 derived from Nephila clavipes, and ADF3 and ADF4 derived from Araneus diadematus. ADF3 is one of the two major dragline silk proteins of Araneus diadematus. Modified spider silk fibroin derived from natural spider silk fibroin may be modified spider silk fibroin derived from these dragline silk proteins. Modified spider silk fibroin derived from ADF3 has excellent characteristics of being relatively easily synthesized and having excellent strength, elongation, and toughness.

A weft thread protein is produced in the flagelliform gland of spiders. Examples of weft thread proteins include a flagelliform silk protein derived from Nephila clavipes.

Modified spider silk fibroin may be recombinant spider silk fibroin. Examples of recombinant spider silk fibroin include mutants, analogs, or derivatives of natural spider silk fibroin. A suitable example of such modified spider silk fibroin is recombinant spider silk fibroin of large spinneret dragline silk proteins. For example, recombinant spider silk fibroin is produced in several heterologous protein production systems. Methods for producing recombinant spider silk fibroin use transgenic goat, transgenic silkworm, or recombinant plants or mammalian cells.

Recombinant spider silk fibroin can be obtained by, for example, deleting one or a plurality of sequences encoding the (A)ₙ motif from the cloned genetic sequence of naturally occurring fibroin. Alternatively, recombinant spider silk fibroin can be obtained by, for example, designing an amino acid sequence corresponding to an amino acid sequence in which one or a plurality of (A)ₙ motifs have been deleted from the amino acid sequence of naturally-occurring fibroin, and chemically synthesizing a nucleic acid encoding the designed amino acid sequence. In any case, modification on the amino acid sequence corresponding to substitution, deletion, insertion, and/or addition of one or a plurality of amino acid residues may be further performed in addition to the modification corresponding to deletion of the (A)ₙ motifs from the amino acid sequence of naturally-occurring fibroin. Substitution, deletion, insertion, and/or addition of amino acid residues can be carried out by methods well known to those skilled in the art, such as site-directed mutagenesis. Specifically, they can be carried out according to a method described in documents such as Nucleic Acid Res. 10, 6487 (1982), and Methods in Enzymology, 100, 448 (1983).

Examples of recombinant spider silk fibroin of large spinneret dragline silk proteins and modified spider silk fibroin derived from silkworm silk include a protein including a domain sequence represented by Formula 1: [(A)ₙ motif-REP]ₘ. In the (A)ₙ motif in Formula 1, A represents an alanine residue, and n may be an integer of 2 to 27, 2 to 20, 4 to 27, 4 to 20, 8 to 20, 10 to 20, 4 to 16, 8 to 16, or 10 to 16. It is sufficient that the ratio of the number of alanine residues be 40% or more with respect to the total number of amino acid residues in the (A)ₙ motif, and the ratio may be 60% or more, 70% or more, 80% or more, 83% or more, 85% or more, 86% or more, 90% or more, 95% or more, or 100% (which means that the (A)n motif consists of only alanine residues). REP represents an amino acid sequence consisting of 2 to 200 amino acid residues. m represents an integer of 2 to 300. The total number of glycine (Gly) residues, serine (Ser) residues, and alanine (Ala) residues contained in the amino acid sequence represented by Formula 1 is preferably 40% or more and may be 60% or more or 70% or more, with respect to the total number of amino acid residues. The plurality of (A)ₙ motifs may be the same amino acid sequences or different amino acid sequences. A plurality of REP's may be the same amino acid sequences or different amino acid sequences. Specific examples of modified spider silk fibroin derived from large spinneret dragline silk include a protein including the amino acid sequence set forth in SEQ ID NO: 1 or SEQ ID NO: 2.

Examples of fibroin derived from weft thread proteins include a protein including a domain sequence represented by Formula 2: [REP2]ₒ (in Formula 2, REP2 represents an amino acid sequence consisting of Gly-Pro-Gly-Gly-X, and X represents one amino acid selected from the group consisting of Alanine (Ala), Serine (Ser), Tyrosine (Tyr), and Valine (Val). o represents an integer of 8 to 300). Examples of fibroin derived from weft thread proteins include a protein including 10 or more, preferably 20 or more, more preferably 30 or more units of the amino acid sequence represented by Formula 2: REP2. For recombinant protein production using a microorganism such as Escherichia coli as a host, fibroin derived from weft thread proteins may have a molecular weight of 500 kDa or less, 300 kDa or less, or 200 kDa or less from the viewpoint of productivity. Specific examples include a protein including the amino acid sequence set forth in SEQ ID NO: 3. The amino acid sequence set forth in SEQ ID NO: 3 is an amino acid sequence obtained by combining an amino acid sequence (referred to as a PR1 sequence) from the 1220th residue to the 1659th residue from the N-terminus, which corresponds to a repeat moiety and motif of a partial sequence (NCBI Accession No: AAF36090, GI: 7106224) of the flagelliform silk protein of Nephila clavipes obtained from the NCBI database, with a C-terminal amino acid sequence from the 816th residue to the 907th residue from the C-terminus of a partial sequence (NCBI Accession No: AAC38847, GI: 2833649) of the flagelliform silk protein of Nephila clavipes obtained from the NCBI database, and then adding the amino acid sequence (tag sequence and hinge sequence) set forth in SEQ ID NO: 4 to the N-terminus of the combined sequence.

Fibroin can be produced by, for example, expressing a nucleic acid in a host transformed with an expression vector having a nucleic acid sequence encoding a desired protein and one or a plurality of regulatory sequences operably linked to the nucleic acid sequence.

A method for manufacturing a nucleic acid encoding desired fibroin is not particularly limited. The nucleic acid can be manufactured by, for example, a method in which a gene encoding natural fibroin is amplified and cloned by polymerase chain reaction (PCR) or the like; or a method of chemically synthesizing a nucleic acid. A method for chemically synthesizing a nucleic acid is not particularly limited and, for example, genes can be chemically synthesized by a method of linking, by PCR or the like, oligonucleotides that are automatically synthesized by AKTA oligopilot plus 10/100 (GE Healthcare Japan Ltd.) or the like, based on amino acid sequence information of structural proteins obtained from the NCBI web database and the like. In this case, in order to facilitate purification and/or confirmation of proteins, a nucleic acid encoding a protein consisting of an amino acid sequence obtained by adding an amino acid sequence consisting of a start codon and a His10 tag to the N-terminus of the above amino acid sequence may be synthesized.

### Examples

Hereinafter, the present invention will be described more specifically with reference to examples. However, the present invention is not limited to the following examples.

### 1. Manufacture of Modified Spider Silk Fibroin

### (1) Production of Plasmid Expression Strain

Modified spider silk fibroin (hereinafter, also referred to as "PRT799") having the amino acid sequence set forth in SEQ ID NO: 5 was designed on the basis of the base sequence and amino acid sequence of fibroin (GenBank Accession No: P46804.1, GI: 1174415) derived from Nephila clavipes. The amino acid sequence set forth in SEQ ID NO: 5 has an amino acid sequence obtained by subjecting the amino acid sequence of fibroin derived from Nephila clavipes to substitution, insertion, and deletion of amino acid residues for the purpose of improving productivity and the amid acid sequence (tag sequence and hinge sequence) set forth in SEQ ID NO: 6 added to the N-terminus of the above amino acid sequence. Modified spider silk fibroin (hereinafter, also referred to as "PRT966") having the amino acid sequence set forth in SEQ ID NO: 7 was also designed.

A nucleic acid encoding PRT799 or PRT966 was synthesized. In the nucleic acid, an NdeI site was added to the 5' end and an EcoRI site was added downstream of the stop codon. The nucleic acid was cloned into a cloning vector (pUC118). Thereafter, the same nucleic acid was cleaved by a restriction enzyme treatment with NdeI and EcoRI, and then recombined into a protein expression vector pET-22b(+) to obtain an expression vector.

### (2) Expression of Protein

Escherichia coli BLR (DE3) was transformed with the obtained expression vector pET-22b(+). The transformed Escherichia coli was cultured in 2 mL of an LB medium containing ampicillin for 15 hours. The culture solution was added to 100 mL of a seed culture medium (Table 1) containing ampicillin so that the OD600 was 0.005. The temperature of the culture solution was maintained at 30°C, and flask culture was carried out (for about 15 hours) until the OD600 reached 5, thereby obtaining a seed culture solution.

**[Table 1]**

| Seed Culture Medium | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

The seed culture solution was added to a jar fermenter containing 500 mL of a production medium (Table 2) so that the OD600 was 0.05. The transformed Escherichia coli was cultured while the temperature of the culture solution was maintained at 37°C. The culture solution was maintained at pH 6.9, and the concentration of oxygen dissolved in the culture solution was maintained at 20% of the saturated concentration of dissolved oxygen.

**[Table 2]**

| Production Medium | |
|---|---|
| Reagent | Concentration (g/L) |
| Glucose | 12.0 |
| KH₂PO₄ | 9.0 |
| MgSO₄·7H₂O | 2.4 |
| Yeast Extract | 15 |
| FeSO₄·7H₂O | 0.04 |
| MnSO₄·5H₂O | 0.04 |
| CaCl₂·2H₂O | 0.04 |
| GD-113 (Defoamer) | 0.1 (mL/L) |

Immediately after glucose in the production medium was completely consumed, a feed solution (455 g/1L of glucose and 120 g/1L of Yeast Extract) was added at a rate of 1 mL/min. Thereafter, the culture was continued at pH 6.9 for 20 hours while the temperature of the culture solution was maintained at 37°C. The concentration of oxygen dissolved in the culture solution was maintained at 20% of the saturated concentration of dissolved oxygen.

Thereafter, 1 M isopropyl-β-thiogalactopyranoside (IPTG) was added to the culture solution so that the final concentration became 1 mM to induce the expression of the target protein. Twenty hours after addition of IPTG, the culture solution was centrifuged to recover the bacterial cells. SDS-PAGE was carried out using the bacterial cells prepared from the culture solution before the addition of IPTG and after the addition of IPTG. The expression of the target modified spider silk fibroin "PRT799" or "PRT966" was confirmed by a band having a size of the target spider silk fibroin appeared according to the addition of IPTG.

### (3) Purification of Protein

The bacterial cells recovered 2 hours after the addition of IPTG were washed with 20 mM Tris-HCl buffer (pH 7.4). The bacterial cells after washing were suspended in 20 mM Tris-HCl buffer solution (pH 7.4) containing about 1 mM PMSF, and the cells were disrupted with a high-pressure homogenizer (available from GEA Niro Soavi SpA). The disrupted cells were centrifuged to obtain a precipitate. The obtained precipitate was washed with 20 mM Tris-HCl buffer solution (pH 7.4) until reaching a high purity. The precipitate after washing was suspended in 8 M guanidine buffer solution (8 M guanidine hydrochloride, 10 mM sodium dihydrogen phosphate, 20 mM NaCl, 1 mM Tris-HCl, pH 7.0) so that the concentration became 100 mg/mL. The suspension was stirred with a stirrer at 60°C for 30 minutes so that the precipitate was dissolved. After dissolution, dialysis was carried out with water using a dialysis tube (cellulose tube 36/32 available from Sanko Junyaku Co., Ltd). The white aggregated protein obtained after dialysis was recovered by centrifugation. The recovered aggregated protein was dried with a freeze dryer to obtain a protein powder containing modified spider silk fibroin "PRT799" or "PRT966".

### 2. Fibroin Solution and Protein Fiber

### 2-1. PRT799

### (Example 1-1)

### Dispersion Step

A mixing device (flow jet mixer MW-J-300-S available from Funken Powtechs, Inc) having a disc-shaped rotary body for mixing was provided. With the rotary body of the mixing device rotating at a rotational speed of 900 rpm, a dissolving liquid at 20°C containing DMSO and 4% by mass of lithium chloride was continuously supplied onto the rotation surface of the rotary body, whereby a thin film of the dissolving liquid was flowed on the rotation surface. The protein powder containing PRT799 was supplied through a powder inlet above the rotation surface and introduced to the dissolving liquid on the rotation surface. The dissolving liquid was supplied at a flow rate of 15 kg/hr by using a gear pump. The protein powder was supplied at a rate of 5 kg/hr by using a feeder. Accordingly, the flowing dissolving liquid and the protein powder were continuously mixed to form a slurry containing these.

### Dissolution Step

The obtained slurry was fed to an in-line mixer (static mixer available from Noritake Co., Ltd.: model SMHEDM-25A(24)/SL) maintained at 80°C by using a progressing cavity pump. The flow rate of the slurry was 0.3 L/min. Twenty four static mixer elements were previously attached to the in-line mixer. The slurry was stirred and heated in the in-line mixer, whereby the protein powder was dissolved in the dissolving liquid. The time it took for the slurry to pass through the in-line mixer was 1 minute, and the temperature of the fibroin solution discharged from the in-line mixer was 70°C.

### Filtration, Defoaming, and Cooling

The obtained fibroin solution was filtered through a sintered metal filter with 1 µm mesh, whereby insoluble matter was removed. Next, the fibroin solution was defoamed by maintaining the fibroin solution under a reduced pressure of -0.1 MPa for 60 minutes. Subsequently, the fibroin solution was cooled to 15°C by feeding the fibroin solution to an in-line mixer (the same model as descried above) maintained at 10°C. The cooled fibroin solution was fed to a storage tank.

### Spinning (Protein Fiber)

A filament containing modified spider silk fibroin was obtained by dry-wet-type spinning using the cooled fibroin solution as a spinning raw liquid. The conditions of dry-wet-type spinning are as described below.
- Temperature of coagulation liquid (methanol): 5°C to 10°C
- Total draw ratio: 5 times
- Drying temperature: 80°C

### (Example 1-2)

A fibroin solution was prepared through the same dispersion step and the same dissolution step as in Example 1-1 except that formic acid was used as a dissolving liquid instead of the dissolving liquid containing DMSO and 4% by mass of lithium chloride. A filament was produced by using the obtained fibroin solution in the same manner as in Example 1-1.

### (Example 1-3)

A fibroin solution was prepared in the same manner as in Example 1-1 except that the number of static mixer elements used in the dissolution step was changed to 10. A filament was produced by using the obtained fibroin solution in the same manner as in Example 1-1.

### (Example 1-4)

A slurry obtained in the same dispersion step as in Example 1-1 was supplied to a 100-L tank with a paddle-type stirring blade. Hot water at 80°C was flowed through a tank jacket. The protein powder was dissolved in the dissolving liquid by heating the slurry for 60 minutes under stirring with the stirring blade to obtain a fibroin solution. A filament was produced by using the obtained fibroin solution in the same manner as in Example 1-1.

### (Comparative Example 1-1)

To a 100-L tank with a paddle-type stirring blade, 16 kg of protein powder and 64 kg of a dissolving liquid containing DMSO and 4% by mass of lithium chloride were added. These were stirred in the tank for 60 minutes to obtain a slurry. A fibroin solution was obtained by using the obtained slurry in the same dissolution step as in Example 1-1. A filament was produced by using the obtained fibroin solution in the same manner as in Example 1-1.

### (Comparative Example 1-2)

A slurry was formed in the tank in the same manner as in Comparative Example 1. Subsequently, hot water at 80°C was flowed through the tank jacket, and the protein powder was dissolved in the dissolving liquid by heating the slurry for 60 minutes under stirring to obtain a fibroin solution. A filament was produced by using the obtained fibroin solution in the same manner as in Example 1-1.

### 2-2. PRT966

### (Example 2-1)

A slurry containing a protein powder and a dissolving liquid was formed in the same dispersion step as in Example 1-1, except that a protein powder containing PRT966 was used instead of the protein powder containing PRT799, and formic acid was used as a dissolving liquid. The supply of the dissolving liquid and formic acid was adjusted such that the concentration of protein in the slurry was 31% by mass based on the mass of the slurry.

The obtained slurry was fed to an in-line mixer (static mixer available from Noritake Co., Ltd.: model SMHEDM-25A(24)/SL) maintained at 80°C by using a progressing cavity pump. The flow rate of the slurry was 0.3 L/min. Twenty four static mixer elements were previously attached to the in-line mixer. The slurry was stirred and heated in the in-line mixer, whereby the protein powder was dissolved in the dissolving liquid. The time it took for the slurry to pass through the in-line mixer was 1 minute, and the temperature of the fibroin solution discharged from the in-line mixer was 70°C.

A filament was produced by using the obtained fibroin solution in the same manner as in Example 1-1.

### (Example 2-2)

A slurry obtained in the same dispersion step as in Example 2-1 was supplied to a 100-L tank with a paddle-type stirring blade. Hot water at 40°C was flowed through a tank jacket. The protein powder was dissolved in the dissolving liquid by heating the slurry for 60 minutes under stirring with the stirring blade to obtain a fibroin solution. A filament was produced by using the obtained fibroin solution in the same manner as in Example 1-1.

### (Comparative Example 2-1)

To a 100-L tank with a paddle-type stirring blade, 31 kg of protein powder and 69 kg of formic acid were added. These were stirred in the tank for 60 minutes to obtain a slurry. A fibroin solution was obtained by using the obtained slurry in the same dissolution step using the in-line mixer as in Example 2-1. A filament was produced by using the obtained fibroin solution in the same manner as in Example 1-1.

### (Comparative Example 2-2)

A slurry was formed in the tank in the same manner as in Comparative Example 2-1. Subsequently, hot water at 40°C was flowed through the tank jacket, and the protein powder was dissolved in the dissolving liquid by heating the slurry for 60 minutes under stirring to obtain a fibroin solution. A filament was produced by using the obtained fibroin solution in the same manner as in Example 1-1.

### 3. Evaluation

### (1) Rate of Lump Generation

A slurry (1000 g) was placed in a container having a volume of 1 L and including a sintered metal filter with 2 mm mesh in a lower part. The slurry was passed through the filter by pressuring the container with 0.05 MPa nitrogen. Lumps in the slurry did not pass through the filter. The mass W₁ (g) of the slurry that had passed through the filter was measured. The rate of lump generation was calculated from Formula: the rate of lump generation = {(1000 - W)₁/1000} × 100.

### (2) Dissolution State (Filtration Rate)

The dissolution state of the protein powder in the fibroin solution before the fibroin solution passing through a sintered metal filter was evaluated on the basis of the filtration rate. The fibroin solution (1000 g) was filtered through a sintered metal filter with 4.7 cm² filter area and 1 µm mesh by feeding the fibroin solution by using a metering pump while maintaining the fibroin solution at 40°C. The mass W₂ (g) of the fibroin solution that had passed through the filter was measured. The filtration rate was calculated from Formula: the filtration rate = (W₂/1000) × 100. A large filtration rate means a small amount of undissolved fibroin.

### (3) Viscosity of Fibroin Solution

The viscosity of the fibroin solution after cooling was measured by using a viscometer (model EMS-01S available from Kyoto Electronics Manufacturing Co., Ltd). The measurement conditions are as described below.
- Temperature: 40°C
- Measurement time: 2 minutes
- Probe rotation speed: 2000 rpm

### (4) Strength and Elongation of Fiber

The opposite ends of the filament were fixed to a test strip with an adhesive, and tensile testing was carried out by using a tensile tester (available from Instron Co., Ltd., model: 3342) under the conditions of a gripping jig distance of 20 mm and a tensile speed of 10 cm/min. Tensile testing was carried out in an environment at a temperature of 20°C and a relative humidity of 65%. The strength (stress at break) and the elongation (elongation at break) were determined from the obtained stress-strain curve. The strength and elongation shown in Table 3 are relative values based on Comparative Example 1-2. The strength and elongation shown in Table 4 are relative values based on Comparative Example 2-2.

**[Table 3]**

| | | Example 1-1 | Example 1-2 | Example 1-3 | Example 1-4 | Comparative Example 1-1 | Comparative Example 1-2 |
|---|---|---|---|---|---|---|---|
| Modified Fibroin | | PRT799 | PRT799 | PRT799 | PRT799 | PRT799 | PRT799 |
| Solvent | | DMSO | formic acid | DMSO | DMSO | DMSO | DMSO |
| Dispersion Step | | thin film | thin film | thin film | thin film | stirring in tank | stirring in tank |
| Dissolution Step | Device | in-line mixer | in-line mixer | in-line mixer | stirring in tank | in-line mixer | stirring in tank |
| | Number of Elements | 24 | 24 | 10 | | 24 | |
| | Time | 1 min | 1 min | 1 min | 60 min | 1 min | 60 min |
| Rate of Lump Generation [% by mass] | | 0 | 0 | 0 | 0 | 18 | 24 |
| Dissolution State (Filtration Rate, [% by mass]) | | 99 | 99 | 99 | 98 | 76 | 71 |
| Solution Viscosity [mPa·s] | | 25000 | 15000 | 24000 | 21000 | 16000 | 9000 |
| Fiber | Strength | 167% | 213% | 160% | 133% | 120% | 100% |
| | Elongation | 194% | 83% | 194% | 106% | 183% | 100% |

**[Table 4]**

| | | Example 2-1 | Example 2-2 | Comparative Example 2-1 | Comparative Example 2-2 |
|---|---|---|---|---|---|
| Modified Fibroin | | PRT966 | PRT966 | PRT966 | PRT966 |
| Solvent | | formic acid | formic acid | formic acid | formic acid |
| Dispersion Step | | thin film | thin film | stirring in tank | stirring in tank |
| Dissolution Step | Device | in-line mixer | stirring in tank | in-line mixer | stirring in tank |
| | Number of Elements | 24 | | 24 | |
| | Time | 1 min | 60 min | 1 min | 60 min |
| Rate of Lump Generation [% by mass] | | 0 | 1 | 20 | 28 |
| Dissolution State (Filtration Rate, [% by mass]) | | 99 | 97 | 73 | 66 |
| Solution Viscosity [mPa·s] | | 40900 | 38900 | 29200 | 18800 |
| Draw Ratio | | 7 times | 6.5 times | 6.2 times | 6 times |
| Fiber | Strength | 123% | 120% | 118% | 100% |
| | Elongation | 54% | 87% | 110% | 100% |

The evaluation results shown in Table 3 and Table 4 indicate that generation of lumps in forming the slurry is sufficiently suppressed according to the methods of Examples including the dispersion step for continuously forming the slurry while flowing the thin film of the dissolving liquid. In each Example, the fibroin solution with less undissolved matter was easily obtained by dissolving, in the solvent, the protein powder in the slurry. In addition, the protein fiber having good mechanical properties was formed from the fibroin solution of each Example. The mechanical properties of the protein fiber were particularly preferable when the protein fiber was manufactured by using the fibroin solutions of Examples 1-1 to 1-3 in which the dispersion step for forming the slurry while flowing the thin film of the dissolving liquid is combined with the dissolution step in the in-line mixer.

For the fiber of PRT966, the elongation in Examples 2-1 and 2-2 tends to be smaller than that in Comparative Examples 2-1 and 2-2, which mainly results from the draw ratio being relatively larger in Examples 2-1 and 2-2. In Examples 2-1 and 2-2, the drawability increases as the solubility of fibroin increases, which results in a high draw ratio. With a high draw ratio, a fiber having a higher strength can be obtained. A fiber having a higher elongation may be obtained by setting the draw ratio to a lower value in Examples 2-1 and 2-2.

### Reference Signs List

- 1: Protein powder
- 3: Dissolving liquid
- 3a: Thin film
- 5: Slurry
- 7: Fibroin solution
- 10: Rotary body
- 11: Shaft
- 12: Controller
- 20: Mixing device
- 21: Dissolving liquid inlet
- 23: Feed pipe
- 25: Retention tank
- 25A: Bottom surface
- 25B: Inner circumferential surface
- 30: Powder inlet
- 40: In-line mixer
- 41: Pipe
- 43: Static mixer element
- 45: Jacket
- 45a: Heating medium inlet
- 45b: Heating medium outlet
- S: Rotation surface

## Claims

1. A method for manufacturing a fibroin solution, the method comprising:
a step of forming a slurry containing a solvent-containing dissolving liquid and a fibroin-containing protein powder dispersed in the dissolving liquid by continuously introducing the protein powder to a thin film of the dissolving liquid while flowing the thin film; and
a step of forming a fibroin solution by dissolving, in the dissolving liquid, the protein powder in the slurry.

2. The method according to claim 1, wherein the thin film of the dissolving liquid is flowed on a surface by continuously supplying the dissolving liquid onto the surface.

3. The method according to claim 1, wherein the step of forming the slurry includes
continuously supplying the dissolving liquid onto a rotation surface of a rotary body by using a metering pump so that the thin film of the dissolving liquid flows on the rotation surface, and
continuously supplying the protein powder by using a metering feeder so that the protein powder is continuously introduced to the thin film on the rotation surface.

4. The method according to any one of claims 1 to 3, wherein the protein powder in the slurry is dissolved in the dissolving liquid by heating the slurry under stirring in an in-line mixer.

5. The method according to any one of claims 1 to 4, further comprising a step of cooling the fibroin solution under stirring in the in-line mixer.

6. The method according to any one of claims 1 to 5, further comprising a step of defoaming the fibroin solution.

7. The method according to any one of claims 1 to 6, further comprising a step of removing insoluble matter by filtering the fibroin solution.

8. The method according to any one of claims 1 to 7, wherein the solvent contains at least one selected from the group consisting of dimethyl sulfoxide, formic acid, and hexafluoroisopropanol.

9. The method according to any one of claims 1 to 8, wherein the dissolving liquid further contains an inorganic salt.

10. The method according to any one of claims 1 to 9, wherein the fibroin is modified fibroin.

11. The method according to claim 10, wherein the modified fibroin is modified spider silk fibroin.

12. A method for manufacturing a protein molded body, the method comprising:
producing a fibroin solution by the method according to any one of claims 1 to 11; and
producing a fibroin-containing protein molded body by molding using the fibroin solution as a raw liquid for molding.

13. The method according to claim 12, wherein the protein molded body is a protein fiber.


### Sequence Listing

SEQUENCE LISTING

<110>  Spiber Inc.

<120>  METHOD FOR PRODUCING FIBROIN SOLUTION

<130>  FP19-0905-00

<150>  JP2018-172728
<151>  2018-09-14

<160>  7

<170>  PatentIn version 3.5

<210>  1
<211>  597
<212>  PRT
<213>  Artificial Sequence

<220>
<223>  Met-PRT313

<400>  1

Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Ala Ala Ala Ala Ala Gly Gly Tyr Gly Pro Gly Gly Gln Gly
        35                  40                  45

Pro Gly Gln Gln Gly Pro Gly Ser Ser Ala Ala Ala Ala Ala Gly Pro
    50                  55                  60

Gly Gly Tyr Gly Pro Gly Gly Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gly Tyr Gly Pro Gly Gly Gln Gly Pro Gly Gln Gln
            100                 105                 110

Gly Pro Gly Ser Ser Ala Ala Ala Ala Ala Gly Gly Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gly Tyr Gly Gln Gly Pro Tyr Gly Pro Gly Ala Ser Ala
145                 150                 155                 160

Ala Ala Ala Ala Gly Pro Gly Gly Tyr Gly Pro Gly Gly Gln Gly Pro
                165                 170                 175

Ser Ala Ser Ala Ala Ala Ala Ala Gly Ser Gly Gln Gln Gly Pro Gly
            180                 185                 190

Gly Tyr Gly Pro Tyr Ala Ser Ala Ala Ala Ala Ala Gly Gly Tyr Gly
        195                 200                 205

Ser Gly Pro Gly Gln Gln Gly Pro Tyr Gly Pro Gly Gly Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gly Gln Gly Pro Tyr Gly
                245                 250                 255

Pro Gly Ser Ser Ala Ala Ala Ala Ala Gly Gly Tyr Gly Tyr Gly Pro
            260                 265                 270

Gly Gly Gln Gly Pro Tyr Gly Pro Gly Ala Ser Ala Ala Ala Ala Ala
        275                 280                 285

Gly Gly Asn Gly Pro Gly Ser Gly Gly Tyr Gly Pro Gly Gln Gln Gly
    290                 295                 300

Pro Gly Gly Ser Ala Ala Ala Ala Ala Gly Pro Gly Gly Gln Gly Pro
305                 310                 315                 320

Tyr Gly Pro Gly Ala Ser Ala Ala Ala Ala Ala Gly Gly Tyr Gly Pro
                325                 330                 335

Gly Gly Gln Gly Pro Gly Gly Tyr Gly Pro Gly Ser Ser Ala Ala Ala
            340                 345                 350

Ala Ala Gly Pro Gly Gly Gln Gly Pro Tyr Gly Pro Gly Ser Ser Ala
        355                 360                 365

Ala Ala Ala Ala Gly Gly Tyr Gly Pro Gly Gln Gln Gly Pro Tyr Gly
    370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala

405                 410                 415

Gly Pro Gly Gly Gln Gly Pro Tyr Gly Pro Gly Ala Ser Ala Ala Ala
            420                 425                 430

Ala Ala Gly Pro Gly Gly Tyr Gly Pro Gly Gly Gln Gly Pro Ser Ala
        435                 440                 445

Ser Ala Ala Ala Ala Ala Gly Gly Tyr Gly Ser Gly Pro Gly Gly Tyr
    450                 455                 460

Gly Pro Tyr Gly Pro Gly Gly Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gly Tyr Gly Pro Gly Gln Gln Gly Pro Tyr Gly Pro
            500                 505                 510

Gly Gly Ser Ala Ala Ala Ala Ala Gly Pro Gly Ser Gly Gly Tyr Gly
        515                 520                 525

Pro Gly Ala Ser Ala Ala Ala Ala Ala Gly Gly Asn Gly Pro Gly Ser
    530                 535                 540

Gly Gly Tyr Gly Pro Gly Gln Gln Gly Pro Gly Gly Ser Ala Ala Ala
545                 550                 555                 560

Ala Ala Gly Gly Tyr Gln Gln Gly Pro Gly Gly Gln Gly Pro Tyr Gly
                565                 570                 575

Pro Gly Ala Ser Ala Ala Ala Ala Ala Gly Pro Gly Ser Gly Gln Gln
            580                 585                 590

Gly Pro Gly Ala Ser
        595

<210>  2
<211>  590
<212>  PRT
<213>  Artificial Sequence

<220>
<223>  Met-PRT399

<400>  2

Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Gly Gly Tyr Gly Pro Gly Gly Gln Gly Pro Gly Gln Gln Gly
        35                  40                  45

Pro Gly Ser Ser Ala Ala Ala Ala Ala Gly Pro Gly Gly Tyr Gly Pro
    50                  55                  60

Gly Gly Gln Gly Pro Ser Ala Ser Ala Ala Ala Ala Ala Gly Pro Gly
65                  70                  75                  80

Ser Gly Gln Gln Gly Pro Gly Ala Ser Gly Gly Tyr Gly Pro Gly Gly
                85                  90                  95

Gln Gly Pro Gly Gln Gln Gly Pro Gly Ser Ser Ala Ala Ala Ala Ala
            100                 105                 110

Gly Gly Tyr Gly Ser Gly Pro Gly Gln Gln Gly Pro Tyr Gly Ser Ala
        115                 120                 125

Ala Ala Ala Ala Gly Pro Gly Ser Gly Gly Tyr Gly Gln Gly Pro Tyr
    130                 135                 140

Gly Pro Gly Ala Ser Gly Pro Gly Gly Tyr Gly Pro Gly Gly Gln Gly
145                 150                 155                 160

Pro Ser Ala Ser Ala Ala Ala Ala Ala Gly Ser Gly Gln Gln Gly Pro
                165                 170                 175

Gly Gly Tyr Gly Pro Tyr Ala Ser Ala Ala Ala Ala Ala Gly Gly Tyr
            180                 185                 190

Gly Ser Gly Pro Gly Gln Gln Gly Pro Tyr Gly Pro Gly Gly Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gly Gln Gly Pro Tyr Gly Pro Gly Ser Ser
225                 230                 235                 240

Ala Ala Ala Ala Ala Gly Gly Tyr Gly Tyr Gly Pro Gly Gly Gln Gly
                245                 250                 255

Pro Tyr Gly Pro Gly Ala Ser Gly Gly Asn Gly Pro Gly Ser Gly Gly

            260                 265                 270

Tyr Gly Pro Gly Gln Gln Gly Pro Gly Gly Ser Ala Ala Ala Ala Ala
        275                 280                 285

Gly Pro Gly Gly Gln Gly Pro Tyr Gly Pro Gly Ala Ser Ala Ala Ala
    290                 295                 300

Ala Ala Gly Gly Tyr Gly Pro Gly Gly Gln Gly Pro Gly Gly Tyr Gly
305                 310                 315                 320

Pro Gly Ser Ser Gly Pro Gly Gly Gln Gly Pro Tyr Gly Pro Gly Ser
                325                 330                 335

Ser Ala Ala Ala Ala Ala Gly Gly Tyr Gly Pro Gly Gln Gln Gly Pro
            340                 345                 350

Tyr Gly Pro Gly Gly Ser Ala Ala Ala Ala Ala Gly Gly Tyr Gln Gln
        355                 360                 365

Gly Pro Gly Gly Gln Gly Pro Tyr Gly Pro Gly Ala Ser Gly Pro Gly
    370                 375                 380

Gly Gln Gly Pro Tyr Gly Pro Gly Ala Ser Ala Ala Ala Ala Ala Gly
385                 390                 395                 400

Pro Gly Gly Tyr Gly Pro Gly Gly Gln Gly Pro Ser Ala Ser Ala Ala
                405                 410                 415

Ala Ala Ala Gly Gly Tyr Gly Ser Gly Pro Gly Gly Tyr Gly Pro Tyr
            420                 425                 430

Gly Pro Gly Gly Ser Gly Pro Gly Ser Gly Gln Gln Gly Gln Gly Pro
        435                 440                 445

Tyr Gly Pro Gly Ala Ser Ala Ala Ala Ala Ala Gly Gly Tyr Gly Pro
    450                 455                 460

Gly Gln Gln Gly Pro Tyr Gly Pro Gly Gly Ser Ala Ala Ala Ala Ala
465                 470                 475                 480

Gly Pro Gly Ser Gly Gly Tyr Gly Pro Gly Ala Ser Gly Gly Asn Gly
                485                 490                 495

Pro Gly Ser Gly Gly Tyr Gly Pro Gly Gln Gln Gly Pro Gly Gly Ser
            500                 505                 510

Ala Ala Ala Ala Ala Gly Gly Tyr Gln Gln Gly Pro Gly Gly Gln Gly
        515                 520                 525

Pro Tyr Gly Pro Gly Ala Ser Ala Ala Ala Ala Ala Gly Gly Tyr Gly
    530                 535                 540

Ser Gly Pro Gly Gln Gln Gly Pro Tyr Gly Pro Gly Gly Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  3
<211>  559
<212>  PRT
<213>  Artificial

<220>
<223>  Recombinant spider silk protein Flag_92_short2

<400>  3

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro

130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5

<211>  2375
<212>  PRT
<213>  Artificial Sequence

<220>
<223>  PRT799

<400>  5
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Gly Gln
               20                  25                  30
   Asn Gly Pro Gly Ser Gly Gln Gln Gly Pro Gly Gln Ser Gly Gln Tyr
           35                  40                  45
   Gly Pro Gly Gln Gln Gly Pro Gly Gln Gln Gly Pro Gly Ser Ser Ala
       50                  55                  60
   Ala Ala Ala Ala Gly Pro Gly Gln Tyr Gly Pro Gly Gln Gln Gly Pro
   65                  70                  75                  80
   Ser Ala Ser Ala Ala Ala Ala Ala Gly Pro Gly Ser Gly Gln Gln Gly
                   85                  90                  95
   Pro Gly Ala Ser Gly Gln Tyr Gly Pro Gly Gln Gln Gly Pro Gly Gln
               100                 105                 110
   Gln Gly Pro Gly Ser Ser Ala Ala Ala Ala Ala Gly Gln Tyr Gly Ser
           115                 120                 125
   Gly Pro Gly Gln Gln Gly Pro Tyr Gly Ser Ala Ala Ala Ala Ala Gly
       130                 135                 140
   Pro Gly Ser Gly Gln Tyr Gly Gln Gly Pro Tyr Gly Pro Gly Ala Ser
   145                 150                 155                 160
   Gly Pro Gly Gln Tyr Gly Pro Gly Gln Gln Gly Pro Ser Ala Ser Ala
                   165                 170                 175
   Ala Ala Ala Ala Gly Ser Gly Gln Gln Gly Pro Gly Gln Tyr Gly Pro
               180                 185                 190
   Tyr Ala Ser Ala Ala Ala Ala Ala Gly Gln Tyr Gly Ser Gly Pro Gly
           195                 200                 205
   Gln Gln Gly Pro Tyr Gly Pro Gly Gln Ser Gly Ser Gly Gln Gln Gly
       210                 215                 220
   Pro Gly Gln Gln Gly Pro Tyr Ala Ser Ala Ala Ala Ala Ala Gly Pro
   225                 230                 235                 240
   Gly Gln Gln Gly Pro Tyr Gly Pro Gly Ser Ser Ala Ala Ala Ala Ala
                   245                 250                 255
   Gly Gln Tyr Gly Tyr Gly Pro Gly Gln Gln Gly Pro Tyr Gly Pro Gly
               260                 265                 270
   Ala Ser Gly Gln Asn Gly Pro Gly Ser Gly Gln Tyr Gly Pro Gly Gln
           275                 280                 285
   Gln Gly Pro Gly Gln Ser Ala Ala Ala Ala Ala Gly Pro Gly Gln Gln
       290                 295                 300
   Gly Pro Tyr Gly Pro Gly Ala Ser Ala Ala Ala Ala Ala Gly Gln Tyr
   305                 310                 315                 320
   Gly Pro Gly Gln Gln Gly Pro Gly Gln Tyr Gly Pro Gly Ser Ser Gly
                   325                 330                 335
   Pro Gly Gln Gln Gly Pro Tyr Gly Pro Gly Ser Ser Ala Ala Ala Ala
               340                 345                 350
   Ala Gly Gln Tyr Gly Pro Gly Gln Gln Gly Pro Tyr Gly Pro Gly Gln
           355                 360                 365
   Ser Ala Ala Ala Ala Ala Gly Gln Tyr Gln Gln Gly Pro Gly Gln Gln
       370                 375                 380
   Gly Pro Tyr Gly Pro Gly Ala Ser Gly Pro Gly Gln Gln Gly Pro Tyr
   385                 390                 395                 400
   Gly Pro Gly Ala Ser Ala Ala Ala Ala Ala Gly Pro Gly Gln Tyr Gly
                   405                 410                 415
   Pro Gly Gln Gln Gly Pro Ser Ala Ser Ala Ala Ala Ala Ala Gly Gln
               420                 425                 430
   Tyr Gly Ser Gly Pro Gly Gln Tyr Gly Pro Tyr Gly Pro Gly Gln Ser
           435                 440                 445
   Gly Pro Gly Ser Gly Gln Gln Gly Gln Gly Pro Tyr Gly Pro Gly Ala
       450                 455                 460
   Ser Ala Ala Ala Ala Ala Gly Gln Tyr Gly Pro Gly Gln Gln Gly Pro
   465                 470                 475                 480
   Tyr Gly Pro Gly Gln Ser Ala Ala Ala Ala Ala Gly Pro Gly Ser Gly
                   485                 490                 495
   Gln Tyr Gly Pro Gly Ala Ser Gly Gln Asn Gly Pro Gly Ser Gly Gln
               500                 505                 510
   Tyr Gly Pro Gly Gln Gln Gly Pro Gly Gln Ser Ala Ala Ala Ala Ala
           515                 520                 525
   Gly Gln Tyr Gln Gln Gly Pro Gly Gln Gln Gly Pro Tyr Gly Pro Gly
       530                 535                 540
   Ala Ser Ala Ala Ala Ala Ala Gly Gln Tyr Gly Ser Gly Pro Gly Gln
   545                 550                 555                 560
   Gln Gly Pro Tyr Gly Pro Gly Gln Ser Gly Ser Gly Gln Gln Gly Pro
                   565                 570                 575
   Gly Gln Gln Gly Pro Tyr Ala Ser Ala Ala Ala Ala Ala Gly Pro Gly
               580                 585                 590
   Ser Gly Gln Gln Gly Pro Gly Ala Ser Gly Gln Gln Gly Pro Tyr Gly
           595                 600                 605
   Pro Gly Ala Ser Ala Ala Ala Ala Ala Gly Gln Asn Gly Pro Gly Ser
       610                 615                 620
   Gly Gln Gln Gly Pro Gly Gln Ser Gly Gln Tyr Gly Pro Gly Gln Gln
   625                 630                 635                 640
   Gly Pro Gly Gln Gln Gly Pro Gly Ser Ser Ala Ala Ala Ala Ala Gly
                   645                 650                 655
   Pro Gly Gln Tyr Gly Pro Gly Gln Gln Gly Pro Ser Ala Ser Ala Ala
               660                 665                 670
   Ala Ala Ala Gly Pro Gly Ser Gly Gln Gln Gly Pro Gly Ala Ser Gly
           675                 680                 685
   Gln Tyr Gly Pro Gly Gln Gln Gly Pro Gly Gln Gln Gly Pro Gly Ser
       690                 695                 700
   Ser Ala Ala Ala Ala Ala Gly Gln Tyr Gly Ser Gly Pro Gly Gln Gln
   705                 710                 715                 720
   Gly Pro Tyr Gly Ser Ala Ala Ala Ala Ala Gly Pro Gly Ser Gly Gln
                   725                 730                 735
   Tyr Gly Gln Gly Pro Tyr Gly Pro Gly Ala Ser Gly Pro Gly Gln Tyr
               740                 745                 750
   Gly Pro Gly Gln Gln Gly Pro Ser Ala Ser Ala Ala Ala Ala Ala Gly
           755                 760                 765
   Ser Gly Gln Gln Gly Pro Gly Gln Tyr Gly Pro Tyr Ala Ser Ala Ala
       770                 775                 780
   Ala Ala Ala Gly Gln Tyr Gly Ser Gly Pro Gly Gln Gln Gly Pro Tyr
   785                 790                 795                 800
   Gly Pro Gly Gln Ser Gly Ser Gly Gln Gln Gly Pro Gly Gln Gln Gly
                   805                 810                 815
   Pro Tyr Ala Ser Ala Ala Ala Ala Ala Gly Pro Gly Gln Gln Gly Pro
               820                 825                 830
   Tyr Gly Pro Gly Ser Ser Ala Ala Ala Ala Ala Gly Gln Tyr Gly Tyr
           835                 840                 845
   Gly Pro Gly Gln Gln Gly Pro Tyr Gly Pro Gly Ala Ser Gly Gln Asn
       850                 855                 860
   Gly Pro Gly Ser Gly Gln Tyr Gly Pro Gly Gln Gln Gly Pro Gly Gln
   865                 870                 875                 880
   Ser Ala Ala Ala Ala Ala Gly Pro Gly Gln Gln Gly Pro Tyr Gly Pro
                   885                 890                 895
   Gly Ala Ser Ala Ala Ala Ala Ala Gly Gln Tyr Gly Pro Gly Gln Gln
               900                 905                 910
   Gly Pro Gly Gln Tyr Gly Pro Gly Ser Ser Gly Pro Gly Gln Gln Gly
           915                 920                 925
   Pro Tyr Gly Pro Gly Ser Ser Ala Ala Ala Ala Ala Gly Gln Tyr Gly
       930                 935                 940
   Pro Gly Gln Gln Gly Pro Tyr Gly Pro Gly Gln Ser Ala Ala Ala Ala
   945                 950                 955                 960
   Ala Gly Gln Tyr Gln Gln Gly Pro Gly Gln Gln Gly Pro Tyr Gly Pro
                   965                 970                 975
   Gly Ala Ser Gly Pro Gly Gln Gln Gly Pro Tyr Gly Pro Gly Ala Ser
               980                 985                 990
   Ala Ala Ala Ala Ala Gly Pro Gly  Gln Tyr Gly Pro Gly  Gln Gln Gly
           995                 1000                 1005
   Pro Ser  Ala Ser Ala Ala Ala  Ala Ala Gly Gln Tyr  Gly Ser Gly
       1010                 1015                 1020
   Pro Gly  Gln Tyr Gly Pro Tyr  Gly Pro Gly Gln Ser  Gly Pro Gly
       1025                 1030                 1035
   Ser Gly  Gln Gln Gly Gln Gly  Pro Tyr Gly Pro Gly  Ala Ser Ala
       1040                 1045                 1050
   Ala Ala  Ala Ala Gly Gln Tyr  Gly Pro Gly Gln Gln  Gly Pro Tyr
       1055                 1060                 1065
   Gly Pro  Gly Gln Ser Ala Ala  Ala Ala Ala Gly Pro  Gly Ser Gly
       1070                 1075                 1080
   Gln Tyr  Gly Pro Gly Ala Ser  Gly Gln Asn Gly Pro  Gly Ser Gly
       1085                 1090                 1095
   Gln Tyr  Gly Pro Gly Gln Gln  Gly Pro Gly Gln Ser  Ala Ala Ala
       1100                 1105                 1110
   Ala Ala  Gly Gln Tyr Gln Gln  Gly Pro Gly Gln Gln  Gly Pro Tyr
       1115                 1120                 1125
   Gly Pro  Gly Ala Ser Ala Ala  Ala Ala Ala Gly Gln  Tyr Gly Ser
       1130                 1135                 1140
   Gly Pro  Gly Gln Gln Gly Pro  Tyr Gly Pro Gly Gln  Ser Gly Ser
       1145                 1150                 1155
   Gly Gln  Gln Gly Pro Gly Gln  Gln Gly Pro Tyr Ala  Ser Ala Ala
       1160                 1165                 1170
   Ala Ala  Ala Gly Pro Gly Ser  Gly Gln Gln Gly Pro  Gly Ala Ser
       1175                 1180                 1185
   Gly Gln  Gln Gly Pro Tyr Gly  Pro Gly Ala Ser Ala  Ala Ala Ala
       1190                 1195                 1200
   Ala Gly  Gln Asn Gly Pro Gly  Ser Gly Gln Gln Gly  Pro Gly Gln
       1205                 1210                 1215
   Ser Gly Gln Tyr Gly Pro Gly Gln Gln Gly Pro Gly Gln Gln Gly
   1220 1225 1230
   Pro Gly Ser Ser Ala Ala Ala Ala Ala Gly Pro Gly Gln Tyr Gly
   1235 1240 1245
   Pro Gly Gln Gln Gly Pro Ser Ala Ser Ala Ala Ala Ala Ala Gly
   1250 1255 1260
   Pro Gly Ser Gly Gln Gln Gly Pro Gly Ala Ser Gly Gln Tyr Gly
   1265 1270 1275
   Pro Gly Gln Gln Gly Pro Gly Gln Gln Gly Pro Gly Ser Ser Ala
   1280 1285 1290
   Ala Ala Ala Ala Gly Gln Tyr Gly Ser Gly Pro Gly Gln Gln Gly
   1295 1300 1305
   Pro Tyr Gly Ser Ala Ala Ala Ala Ala Gly Pro Gly Ser Gly Gln
   1310 1315 1320
   Tyr Gly Gln Gly Pro Tyr Gly Pro Gly Ala Ser Gly Pro Gly Gln
   1325 1330 1335
   Tyr Gly Pro Gly Gln Gln Gly Pro Ser Ala Ser Ala Ala Ala Ala
   1340 1345 1350
   Ala Gly Ser Gly Gln Gln Gly Pro Gly Gln Tyr Gly Pro Tyr Ala
   1355 1360 1365
   Ser Ala Ala Ala Ala Ala Gly Gln Tyr Gly Ser Gly Pro Gly Gln
   1370 1375 1380
   Gln Gly Pro Tyr Gly Pro Gly Gln Ser Gly Ser Gly Gln Gln Gly
   1385 1390 1395
   Pro Gly Gln Gln Gly Pro Tyr Ala Ser Ala Ala Ala Ala Ala Gly
   1400 1405 1410
   Pro Gly Gln Gln Gly Pro Tyr Gly Pro Gly Ser Ser Ala Ala Ala
   1415 1420 1425
   Ala Ala Gly Gln Tyr Gly Tyr Gly Pro Gly Gln Gln Gly Pro Tyr
   1430 1435 1440
   Gly Pro Gly Ala Ser Gly Gln Asn Gly Pro Gly Ser Gly Gln Tyr
   1445                    1450                    1455
   Gly Pro  Gly Gln Gln Gly Pro  Gly Gln Ser Ala Ala  Ala Ala Ala
       1460                    1465                    1470
   Gly Pro  Gly Gln Gln Gly Pro  Tyr Gly Pro Gly Ala  Ser Ala Ala
       1475                    1480                    1485
   Ala Ala  Ala Gly Gln Tyr Gly  Pro Gly Gln Gln Gly  Pro Gly Gln
       1490                    1495                    1500
   Tyr Gly  Pro Gly Ser Ser Gly  Pro Gly Gln Gln Gly  Pro Tyr Gly
       1505                    1510                    1515
   Pro Gly  Ser Ser Ala Ala Ala  Ala Ala Gly Gln Tyr  Gly Pro Gly
       1520                    1525                    1530
   Gln Gln  Gly Pro Tyr Gly Pro  Gly Gln Ser Ala Ala  Ala Ala Ala
       1535                    1540                    1545
   Gly Gln  Tyr Gln Gln Gly Pro  Gly Gln Gln Gly Pro  Tyr Gly Pro
       1550                    1555                    1560
   Gly Ala  Ser Gly Pro Gly Gln  Gln Gly Pro Tyr Gly  Pro Gly Ala
       1565                    1570                    1575
   Ser Ala  Ala Ala Ala Ala Gly  Pro Gly Gln Tyr Gly  Pro Gly Gln
       1580                    1585                    1590
   Gln Gly  Pro Ser Ala Ser Ala  Ala Ala Ala Ala Gly  Gln Tyr Gly
       1595                    1600                    1605
   Ser Gly  Pro Gly Gln Tyr Gly  Pro Tyr Gly Pro Gly  Gln Ser Gly
       1610                    1615                    1620
   Pro Gly  Ser Gly Gln Gln Gly  Gln Gly Pro Tyr Gly  Pro Gly Ala
       1625                    1630                    1635
   Ser Ala  Ala Ala Ala Ala Gly  Gln Tyr Gly Pro Gly  Gln Gln Gly
       1640                    1645                    1650
   Pro Tyr  Gly Pro Gly Gln Ser  Ala Ala Ala Ala Ala  Gly Pro Gly
       1655                    1660                    1665
   Ser Gly  Gln Tyr Gly Pro Gly  Ala Ser Gly Gln Asn  Gly Pro Gly
       1670                    1675                    1680
   Ser Gly Gln Tyr Gly Pro Gly Gln Gln Gly Pro Gly Gln Ser Ala
   1685 1690 1695
   Ala Ala Ala Ala Gly Gln Tyr Gln Gln Gly Pro Gly Gln Gln Gly
   1700 1705 1710
   Pro Tyr Gly Pro Gly Ala Ser Ala Ala Ala Ala Ala Gly Gln Tyr
   1715 1720 1725
   Gly Ser Gly Pro Gly Gln Gln Gly Pro Tyr Gly Pro Gly Gln Ser
   1730 1735 1740
   Gly Ser Gly Gln Gln Gly Pro Gly Gln Gln Gly Pro Tyr Ala Ser
   1745 1750 1755
   Ala Ala Ala Ala Ala Gly Pro Gly Ser Gly Gln Gln Gly Pro Gly
   1760 1765 1770
   Ala Ser Gly Gln Gln Gly Pro Tyr Gly Pro Gly Ala Ser Ala Ala
   1775 1780 1785
   Ala Ala Ala Gly Gln Asn Gly Pro Gly Ser Gly Gln Gln Gly Pro
   1790 1795 1800
   Gly Gln Ser Gly Gln Tyr Gly Pro Gly Gln Gln Gly Pro Gly Gln
   1805 1810 1815
   Gln Gly Pro Gly Ser Ser Ala Ala Ala Ala Ala Gly Pro Gly Gln
   1820 1825 1830
   Tyr Gly Pro Gly Gln Gln Gly Pro Ser Ala Ser Ala Ala Ala Ala
   1835 1840 1845
   Ala Gly Pro Gly Ser Gly Gln Gln Gly Pro Gly Ala Ser Gly Gln
   1850 1855 1860
   Tyr Gly Pro Gly Gln Gln Gly Pro Gly Gln Gln Gly Pro Gly Ser
   1865 1870 1875
   Ser Ala Ala Ala Ala Ala Gly Gln Tyr Gly Ser Gly Pro Gly Gln
   1880 1885 1890
   Gln Gly Pro Tyr Gly Ser Ala Ala Ala Ala Ala Gly Pro Gly Ser
   1895 1900 1905
   Gly Gln Tyr Gly Gln Gly Pro Tyr Gly Pro Gly Ala Ser Gly Pro
   1910 1915 1920
   Gly Gln Tyr Gly Pro Gly Gln Gln Gly Pro Ser Ala Ser Ala Ala
   1925 1930 1935
   Ala Ala Ala Gly Ser Gly Gln Gln Gly Pro Gly Gln Tyr Gly Pro
   1940 1945 1950
   Tyr Ala Ser Ala Ala Ala Ala Ala Gly Gln Tyr Gly Ser Gly Pro
   1955 1960 1965
   Gly Gln Gln Gly Pro Tyr Gly Pro Gly Gln Ser Gly Ser Gly Gln
   1970 1975 1980
   Gln Gly Pro Gly Gln Gln Gly Pro Tyr Ala Ser Ala Ala Ala Ala
   1985 1990 1995
   Ala Gly Pro Gly Gln Gln Gly Pro Tyr Gly Pro Gly Ser Ser Ala
   2000 2005 2010
   Ala Ala Ala Ala Gly Gln Tyr Gly Tyr Gly Pro Gly Gln Gln Gly
   2015 2020 2025
   Pro Tyr Gly Pro Gly Ala Ser Gly Gln Asn Gly Pro Gly Ser Gly
   2030 2035 2040
   Gln Tyr Gly Pro Gly Gln Gln Gly Pro Gly Gln Ser Ala Ala Ala
   2045 2050 2055
   Ala Ala Gly Pro Gly Gln Gln Gly Pro Tyr Gly Pro Gly Ala Ser
   2060 2065 2070
   Ala Ala Ala Ala Ala Gly Gln Tyr Gly Pro Gly Gln Gln Gly Pro
   2075 2080 2085
   Gly Gln Tyr Gly Pro Gly Ser Ser Gly Pro Gly Gln Gln Gly Pro
   2090 2095 2100
   Tyr Gly Pro Gly Ser Ser Ala Ala Ala Ala Ala Gly Gln Tyr Gly
   2105 2110 2115
   Pro Gly Gln Gln Gly Pro Tyr Gly Pro Gly Gln Ser Ala Ala Ala
   2120 2125 2130
   Ala Ala Gly Gln Tyr Gln Gln Gly Pro Gly Gln Gln Gly Pro Tyr
   2135 2140 2145
   Gly Pro Gly Ala Ser Gly Pro Gly Gln Gln Gly Pro Tyr Gly Pro
   2150 2155 2160
   Gly Ala Ser Ala Ala Ala Ala Ala Gly Pro Gly Gln Tyr Gly Pro
       2165                2170                2175
   Gly Gln Gln Gly Pro Ser Ala Ser Ala Ala Ala Ala Ala Gly Gln
       2180                2185                2190
   Tyr Gly Ser Gly Pro Gly Gln Tyr Gly Pro Tyr Gly Pro Gly Gln
       2195                2200                2205
   Ser Gly Pro Gly Ser Gly Gln Gln Gly Gln Gly Pro Tyr Gly Pro
       2210                2215                2220
   Gly Ala Ser Ala Ala Ala Ala Ala Gly Gln Tyr Gly Pro Gly Gln
       2225                2230                2235
   Gln Gly Pro Tyr Gly Pro Gly Gln Ser Ala Ala Ala Ala Ala Gly
       2240                2245                2250
   Pro Gly Ser Gly Gln Tyr Gly Pro Gly Ala Ser Gly Gln Asn Gly
       2255                2260                2265
   Pro Gly Ser Gly Gln Tyr Gly Pro Gly Gln Gln Gly Pro Gly Gln
       2270                2275                2280
   Ser Ala Ala Ala Ala Ala Gly Gln Tyr Gln Gln Gly Pro Gly Gln
       2285                2290                2295
   Gln Gly Pro Tyr Gly Pro Gly Ala Ser Ala Ala Ala Ala Ala Gly
       2300                2305                2310
   Gln Tyr Gly Ser Gly Pro Gly Gln Gln Gly Pro Tyr Gly Pro Gly
       2315                2320                2325
   Gln Ser Gly Ser Gly Gln Gln Gly Pro Gly Gln Gln Gly Pro Tyr
       2330                2335                2340
   Ala Ser Ala Ala Ala Ala Ala Gly Pro Gly Ser Gly Gln Gln Gly
       2345                2350                2355
   Ser Ser Val Asp Lys Leu Ala Ala Ala Leu Glu His His His His
       2360                2365                2370
   His His
       2375

<210>  6

<211>  12
<212>  PRT
<213>  Artificial Sequence

<220>
<223>  His6Tag plus Hinge

<400>  6

Met His His His His His His Ser Ser Gly Ser Ser
1               5                   10


<210>  7
<211>  1190
<212>  PRT
<213>  Artificial Sequence

<220>
<223>  PRT966

<400>  7

Met His His His His His His Ser Ser Gly Ser Ser Gly Pro Gly Val
1               5                   10                  15


Phe Gly Pro Tyr Gly Pro Gly Ala Ser Ala Ala Ala Ala Ala Gly Ile
            20                  25                  30


Asn Gly Pro Gly Ser Gly Val Phe Gly Pro Gly Ile Ser Gly Ile Tyr
        35                  40                  45


Gly Pro Gly Val Phe Gly Pro Gly Val Phe Gly Pro Gly Ser Ser Ala
    50                  55                  60


Ala Ala Ala Ala Gly Pro Gly Ile Tyr Gly Pro Gly Val Phe Gly Pro
65                  70                  75                  80


Ser Ala Ser Ala Ala Ala Ala Ala Gly Pro Gly Ser Gly Val Phe Gly
                85                  90                  95


Pro Gly Ala Ser Gly Ile Tyr Gly Pro Gly Val Phe Gly Pro Gly Val
            100                 105                 110


Phe Gly Pro Gly Ser Ser Ala Ala Ala Ala Ala Gly Ile Tyr Gly Ser
        115                 120                 125


Gly Pro Gly Val Phe Gly Pro Tyr Gly Ser Ala Ala Ala Ala Ala Gly
    130                 135                 140


Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Phe Gly Pro Ser Ala Ser Ala
                165                 170                 175

Ala Ala Ala Ala Gly Ser Gly Val Phe Gly Pro Gly Ile Tyr Gly Pro
            180                 185                 190

Tyr Ala Ser Ala Ala Ala Ala Ala Gly Ile Tyr Gly Ser Gly Pro Gly
        195                 200                 205

Val Phe Gly Pro Tyr Gly Pro Gly Ile Ser Gly Ser Gly Val Phe Gly
    210                 215                 220

Pro Gly Val Phe Gly Pro Tyr Ala Ser Ala Ala Ala Ala Ala Gly Pro
225                 230                 235                 240

Gly Val Phe Gly Pro Tyr Gly Pro Gly Ser Ser Ala Ala Ala Ala Ala
                245                 250                 255

Gly Ile Tyr Gly Tyr Gly Pro Gly Val Phe Gly Pro Tyr Gly Pro Gly
            260                 265                 270

Ala Ser Gly Ile Asn Gly Pro Gly Ser Gly Ile Tyr Gly Pro Gly Val
        275                 280                 285

Phe Gly Pro Gly Ile Ser Ala Ala Ala Ala Ala Gly Pro Gly Val Phe
    290                 295                 300

Gly Pro Tyr Gly Pro Gly Ala Ser Ala Ala Ala Ala Ala Gly Ile Tyr
305                 310                 315                 320

Gly Pro Gly Val Phe Gly Pro Gly Ile Tyr Gly Pro Gly Ser Ser Gly
                325                 330                 335

Pro Gly Val Phe Gly Pro Tyr Gly Pro Gly Ser Ser Ala Ala Ala Ala
            340                 345                 350

Ala Gly Ile Tyr Gly Pro Gly Val Phe Gly Pro Tyr Gly Pro Gly Ile
        355                 360                 365

Ser Ala Ala Ala Ala Ala Gly Ile Tyr Val Phe Gly Pro Gly Val Phe
    370                 375                 380

Gly Pro Tyr Gly Pro Gly Ala Ser Gly Pro Gly Val Phe Gly Pro Tyr
385                 390                 395                 400

Gly Pro Gly Ala Ser Ala Ala Ala Ala Ala Gly Pro Gly Ile Tyr Gly
                405                 410                 415

Pro Gly Val Phe Gly Pro Ser Ala Ser Ala Ala Ala Ala Ala Gly Ile
        420                 425                 430

Tyr Gly Ser Gly Pro Gly Ile Tyr Gly Pro Tyr Gly Pro Gly Ile Ser
    435                 440                 445

Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
        515                 520                 525

Gly Ile Tyr Val Phe Gly Pro Gly Val Phe Gly Pro Tyr Gly Pro Gly
    530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
                565                 570                 575

Gly Val Phe Gly Pro Tyr Ala Ser Ala Ala Ala Ala Ala Gly Pro Gly
            580                 585                 590

Ser Gly Val Phe Gly Pro Gly Ala Ser Gly Pro Gly Val Phe Gly Pro
        595                 600                 605

Tyr Gly Pro Gly Ala Ser Ala Ala Ala Ala Ala Gly Ile Asn Gly Pro
    610                 615                 620

Gly Ser Gly Val Phe Gly Pro Gly Ile Ser Gly Ile Tyr Gly Pro Gly
625                 630                 635                 640

Val Phe Gly Pro Gly Val Phe Gly Pro Gly Ser Ser Ala Ala Ala Ala
                645                 650                 655

Ala Gly Pro Gly Ile Tyr Gly Pro Gly Val Phe Gly Pro Ser Ala Ser
            660                 665                 670

Ala Ala Ala Ala Ala Gly Pro Gly Ser Gly Val Phe Gly Pro Gly Ala
        675                 680                 685

Ser Gly Ile Tyr Gly Pro Gly Val Phe Gly Pro Gly Val Phe Gly Pro
    690                 695                 700

Gly Ser Ser Ala Ala Ala Ala Ala Gly Ile Tyr Gly Ser Gly Pro Gly
705                 710                 715                 720

Val Phe Gly Pro Tyr Gly Ser Ala Ala Ala Ala Ala Gly Pro Gly Ser
                725                 730                 735

Gly Ile Tyr Gly Ile Gly Pro Tyr Gly Pro Gly Ala Ser Gly Pro Gly
            740                 745                 750

Ile Tyr Gly Pro Gly Val Phe Gly Pro Ser Ala Ser Ala Ala Ala Ala
        755                 760                 765

Ala Gly Ser Gly Val Phe Gly Pro Gly Ile Tyr Gly Pro Tyr Ala Ser
    770                 775                 780

Ala Ala Ala Ala Ala Gly Ile Tyr Gly Ser Gly Pro Gly Val Phe Gly
785                 790                 795                 800

Pro Tyr Gly Pro Gly Ile Ser Gly Ser Gly Val Phe Gly Pro Gly Val
                805                 810                 815

Phe Gly Pro Tyr Ala Ser Ala Ala Ala Ala Ala Gly Pro Gly Val Phe
            820                 825                 830

Gly Pro Tyr Gly Pro Gly Ser Ser Ala Ala Ala Ala Ala Gly Ile Tyr
        835                 840                 845

Gly Tyr Gly Pro Gly Val Phe Gly Pro Tyr Gly Pro Gly Ala Ser Gly
    850                 855                 860

Ile Asn Gly Pro Gly Ser Gly Ile Tyr Gly Pro Gly Val Phe Gly Pro
865                 870                 875                 880

Gly Ile Ser Ala Ala Ala Ala Ala Gly Pro Gly Val Phe Gly Pro Tyr
                885                 890                 895

Gly Pro Gly Ala Ser Ala Ala Ala Ala Ala Gly Ile Tyr Gly Pro Gly
            900                 905                 910

Val Phe Gly Pro Gly Ile Tyr Gly Pro Gly Ser Ser Gly Pro Gly Val

915                 920                 925

Phe Gly Pro Tyr Gly Pro Gly Ser Ser Ala Ala Ala Ala Ala Gly Ile
    930                 935                 940

Tyr Gly Pro Gly Val Phe Gly Pro Tyr Gly Pro Gly Ile Ser Ala Ala
945                 950                 955                 960

Ala Ala Ala Gly Ile Tyr Val Phe Gly Pro Gly Val Phe Gly Pro Tyr
                965                 970                 975

Gly Pro Gly Ala Ser Gly Pro Gly Val Phe Gly Pro Tyr Gly Pro Gly
            980                 985                 990

Ala Ser Ala Ala Ala Ala Ala Gly  Pro Gly Ile Tyr Gly  Pro Gly Val
        995                 1000                1005

Phe Gly  Pro Ser Ala Ser Ala  Ala Ala Ala Ala Gly  Ile Tyr Gly
    1010                1015                1020

Ser Gly  Pro Gly Ile Tyr Gly  Pro Tyr Gly Pro Gly  Ile Ser Gly
    1025                1030                1035

Pro Gly  Ser Gly Val Phe Gly  Ile Gly Pro Tyr Gly  Pro Gly Ala
    1040                1045                1050

Ser Ala  Ala Ala Ala Ala Gly  Ile Tyr Gly Pro Gly  Val Phe Gly
    1055                1060                1065

Pro Tyr  Gly Pro Gly Ile Ser  Ala Ala Ala Ala Ala  Gly Pro Gly
    1070                1075                1080

Ser Gly  Ile Tyr Gly Pro Gly  Ala Ser Gly Ile Asn  Gly Pro Gly
    1085                1090                1095

Ser Gly  Ile Tyr Gly Pro Gly  Val Phe Gly Pro Gly  Ile Ser Ala
    1100                1105                1110

Ala Ala  Ala Ala Gly Ile Tyr  Val Phe Gly Pro Gly  Val Phe Gly
    1115                1120                1125

Pro Tyr  Gly Pro Gly Ala Ser  Ala Ala Ala Ala Ala  Gly Ile Tyr
    1130                1135                1140

Gly Ser  Gly Pro Gly Val Phe  Gly Pro Tyr Gly Pro  Gly Ile Ser
    1145                1150                1155

Gly Ser Gly Val Phe Gly Pro Gly Val Phe Gly Pro Tyr Ala Ser
    1160                1165                1170

Ala Ala Ala Ala Ala Gly Pro Gly Ser Gly Val Phe Gly Pro Gly
    1175                1180                1185

Ala Ser
    1190